# EUROPÄISCHE PATENTSCHRIFT

(11) **EP 1 269 983 B1**
(45) Veröffentlichungstag und Bekanntmachung des Hinweises auf die Patenterteilung: **10.09.2003**
(21) Anmeldenummer: 01115131.3
(22) Anmeldetag: 22.06.2001
(51) Int. Cl.: A61K 7/48, A61K 7/32, A01N 31/02

(54) **Verwendung von 1,2-Decandiol gegen Körpergeruch verursachende Keime**
Use of 1,2-Decanediol against germs which cause body malodour
Utilisation du 1,2-décanediol contre des germes responsables d' odeurs corporelles désagréables

(43) Veröffentlichungstag der Anmeldung: 02.01.2003
(73) Patentinhaber: Dragoco Gerberding & Co Aktiengesellschaft, 37603 Holzminden (DE)
(72) Erfinder: Schmaus, Gerhard Dr., 37671 Höster-Bosseborn (DE); Herrmann, Martina Dr., 37603 Holzminden (DE); Joppe, Holger, 37586 Dassel (DE)
(74) Vertreter: Eisenführ, Speiser & Partner

(56) Entgegenhaltungen:
- EP-A- 0 524 548
- WO-A-00/25726
- DE-A- 4 320 744
- DE-A- 19 924 496
- US-A- 3 928 557
- US-A- 6 123 953
- US-A- 6 143 307
- DATABASE WPI Section Ch, Week 197639 Derwent Publications Ltd., London, GB; Class C03, AN 1976-72925X XP002022306 & JP 51 091327 A (KAO SOAP CO LTD), 10. August 1976 (1976-08-10)
- PATENT ABSTRACTS OF JAPAN vol. 2000, no. 02, 29. Februar 2000 (2000-02-29) & JP 11 322591 A (MANDOM CORP;KANKOUSHA:KK; HAYASHIBARA BIOCHEM LAB INC), 24. November 1999 (1999-11-24)

## Beschreibung

Die vorliegende Erfindung betrifft 1,2-Decandiol als Mittel zur Hemmung des Wachstums von Körpergeruch verursachenden Keimen, wobei die Körpergeruch verursachen den Keime ausgewählt sind aus der Gruppe, die aus Corynebacterium xerosis, Staphylococcus epidermidis, Brevibacterium epidermidis und Mischungen aus zwei oder mehr der vorgenannten Keime besteht.

Die menschliche Haut wird von einer Vielzahl verschiedener Bakterien besiedelt. Die meisten dieser Bakterien sind nicht pathogen und für den Geruch der Haut irrelevant. Andere hingegen sind in der Lage, körpereigene Sekrete zu zersetzen, wobei es zu mehr oder minder starkem Körpergeruch (insbesondere Achsel- und Fußgeruch) kommen kann. Zu den Mikroorganismen, welche Körpergeruch verursachen können, zählen insbesondere die in der nachfolgenden Tabelle aufgeführten Spezies.

| Mikroorganismen | Wirkung |
|---|---|
| *Staphylococcus epidermidis* | Achselgeruch; Körpergeruch allgemein |
| *Corynebacterium xerosis* | Achselgeruch |
| *Brevibacterium epidermidis* | Achselgeruch; Fußgeruch |

Bei *Staphylococcus epidermidis, Corynebacterium xerosis* und *Brevibacterium epidermidis* handelt es sich um gram-positive Bakterien.

Körpergeruch lässt sich auf unterschiedliche Weisen bekämpfen. Bei einer ersten Bekämpfungsstrategie wird die mikrobielle Erzeugung von Geruchsstoffen nicht oder nur unwesentlich beeinflusst, aber die gebildeten Geruchsstoffe werden durch Überlagerung mit anderen Duftstoffen maskiert oder sie werden von Absorptionsmitteln absorbiert.

Bei einer alternativen Strategie wird versucht, die Bildung von unangenehmen Geruchsstoffen von vornherein zu verhindern. Dieses Ziel lässt sich zum Einen durch den Einsatz von Antiperspirantien erreichen, d. h. durch den Einsatz von Substanzen, die eine Perspiration unterdrücken oder zumindest stark hemmen. Zum Anderen lässt sich die Bildung von Körpergeruch durch den Einsatz antimikrobieller Wirkstoffe bekämpfen, welche aufgrund ihrer Wirkung die Bakterien, welche zur Zersetzung körpereigener Sekrete und damit zur Bildung der unangenehmen Geruchsstoffe führen, zerstören oder deren Vermehrung zumindest maßgeblich hemmen.

Ein prominentes Beispiel für einen antimikrobiellen Wirkstoff gegen Körpergeruch bildende Keime ist die Substanz Famesol, vgl. hierzu DE 27 28 921 A1 (entspricht US 4,220,665) sowie DE 33 15 058 (entspricht EP 0 126 944). Dem Fachmann sind aber auch eine Reihe weiterer Substanzen bekannt, welche bereits zu dem genannten Zweck eingesetzt werden.

In der kosmetischen Industrie besteht ein ständiger Bedarf an Alternativen zu den bislang bekannten antimikrobiellen Wirkstoffen zur Bekämpfung von Körpergeruch. Das Ziel ist dabei immer eine antimikrobielle Substanz mit folgenden Eigenschaften:
- gute Wirksamkeit gegen die für Körpergeruch (inklusive Achsel- und Fußgeruch) verantwortlichen Mikroorganismen bereits in niedriger, aus dermatologischer Sicht unbedenklicher Konzentration,
- toxikologische Unbedenklichkeit,
- gute Hautverträglichkeit, das heißt keine Erzeugung von Hautirritationen bei Applikation einer Wirkstoffmenge, welche die für Körpergeruch verantwortlichen Mikroorganismen abtötet oder zumindest deren Wachstum hemmt,
- Stabilität, insbesondere in den üblichen kosmetischen Formulieren, sowie
- geringe Herstellkosten, das heißt Herstellbarkeit unter Einsatz von Standardverfahren und/oder ausgehend von Standardprekursoren.

Die Suche nach einer geeigneten (Wirk)substanz, die zumindest die erste und darüber hinaus eine oder mehrere der weiteren genannten Eigenschaften in ausreichendem Maße besitzt, ist dem Kosmetikfachmann dadurch erschwert, dass keine Abhängigkeit zwischen der chemischen Struktur einer Substanz einerseits und ihrer biologischen Aktivität gegenüber bestimmten Keimen (z. B. den oben genannten für Körpergeruch verantwortlichen Keimen) besteht. Im gleichen Maße gibt es keinen vorhersehbaren Zusammenhang zwischen der antimikrobiellen Wirkung, der toxikologischen Unbedenklichkeit, der Hautverträglichkeit und/oder der Stabilität

Angesichts dieser Sachlage war es die Aufgabe der vorliegenden Erfindung, einen antimikrobiellen Wirkstoff anzugeben, der zur Hemmung des Wachstums von Körpergeruch verursachenden Keimen geeignet ist.

Insbesondere sollte der anzugebende Wirkstoff gegen *Corynebacterium xerosis*, *Staphylococcus epidermidis* und/oder *Brevibacterium epidermidis* wirksam sein. Die zum Erreichen der gewünschten mikrobiellen Wirkung topisch zu applizierende Wirkstoffmenge sollte dabei vorzugsweise keine Hautirritationen hervorrufen können.

Die genannten Aufgaben werden durch die Angabe von 1,2-Decandiol als antimikrobieller Wirkstoff gelöst.

Die Erfindung beruht dabei auf der überraschenden Erkenntnis, dass 1,2-Decandiol bereits in äußerst geringen Applikationsmengen (Konzentrationen) eine exzellente Wirkung gegenüber Körpergeruch bildenden Keimen im Allgemeinen und gegenüber *Corynebacterium xerosis, Staphylococcus epidermidis, Brevibacterium epidermidis* und Mischungen von zwei oder mehr der genannten Keime im Speziellen besitzt.

Obwohl sich die Fachwelt bereits recht umfangreich mit der der im Einzelfall mehr oder weniger guten antimikrobiellen Wirkung von 1,2-Diolen befasst hatte, gab es bislang keinen Hinweis auf die hervorragenden Eigenschaften des 1,2-Decandiols gegenüber den genannten speziellen Keimen. Aus der Literatur lässt sich auch keine Tendenz ablesen, die diese spezielle Wirkung nahegelegt hätte.

So sind aus der JP-A-51 91327 zwar bestimmte Hinweise zur bakteriostatischen Wirkung von 1,2-Decandiol gegenüber bestimmten Keimen im Zusammenhang mit der Konservierung von Lebensmitteln und Kosmetika bekannt, jedoch keine antimikrobielle Wirkung gegenüber den für Körpergeruch verantwortlichen Keimen.

Aus der JP-A-11 322591 ist lediglich bekannt, dass bestimmte 1,2-Alkandiole mit einer Kettenlänge von 4 bis 10 C-Atomen eingesetzt werden können, um die Dosis konventioneller antiseptischer Mikrobizide in einer antimikrobiellen Formulierung zu reduzieren. Als bevorzugt werden 1,2-Pentandiol und 1,2-Hexandiol sowie 1,2-Octandiol genannt. Ein Hinweis auf die Wirkung von 1,2-Decandiol gegenüber Keimen, die für die Bildung von Körpergeruch verantwortlich sind, ist in der japanischen Schrift nicht enthalten.

In der FR 2,771,632 A1 ist offenbart, dass bestimmte 1,2-Alkandiole mit einer Kettenlänge von 8 bis 18 C-Atomen im Gemisch mit einer N-Acylaminosäure als Mittel gegen Schuppen eingesetzt werden können. Als bevorzugt wird auch hier 1,2-Octandiol genannt.

In der US 6,123,953 wird ausgeführt, dass unverzweigte 1,2-Alkandiole mit einer Kettenlänge von insgesamt 5 bis 14 C-Atomen und vorzugsweise 6 bis 8 C-Atomen zur topischen Applikation auf die Haut und hierbei insbesondere zur Behandlung von Akne, zur Behandlung von Kapillarschichten, zur Desinfektion kleiner Wunden (Kratzer), zur Desinfektion der Hände von Chirurgen und Kranken sowie zur Desinfektion der Euter von milchgebenden Tieren geeignet sind. Die US 5,123,953 offenbart ferner, dass Formulierungen, die neben einem Alkandiol ein Gel des Glyceryl-Polymethacrylat-Typs enthalten, in niedrigen Konzentration zur Behandlung von Akne, Hautschwierigkeiten, Impetigo, Mikroorganismen basierten Körpergerüchen, "athlete's foot" und dergleichen eingesetzt werden können. Diese Einsetzbarkeit wird dabei auf eine synergistische Wechselwirkung zwischen dem Gel und dem Alkandiol zurückgeführt. Als bevorzugtes Alkandiol wird 1,2-Octandiol angegeben. Als Keime, gegen die die genannten Kombinations-Formulierungen wirksam sind, werden genannt: *Staphylococcus aureus, Streptococcus faecalis, Pseudomonas aeruginosa, Escherichia coli, Lysteria monocytogenis, Propionibacterium acnes* sowie pathogene Keime der Arten *Lysteria, Staphylococcus, Streptococcus* und *Pseudomonas.* Eine Wirkung von 1,2-Decandiol gegenüber Körpergeruch bildenden Keimen wie insbesondere *Corynebacterium xerosis, Staphylococcus epidermidis* und/oder *Brevibacterium epidermidis* ist in der US 6,123,953 nicht offenbart.

Die EP 0 524 548 A1 offenbart bestimmte antimikrobiell wirksame-Gemische, die neben (A) einem antimikrobiell wirksamen aromatischen Alkohol der Formel 1, 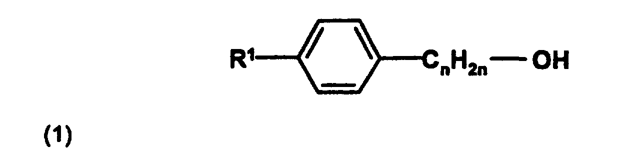 in der R¹ Wasserstoff oder eine Alkylgruppe mit 1 bis 4 C-Atomen und n eine ganze Zahl von 1 bis 6 ist, (B) ein antimikrobiell wirksames 1,2- oder 1,3-Diol der Formel R²-CHOH-(CHR³)ₓ-CH₂OH enthalten, wobei x = 0 oder 1 ist und wenn x = 0 ist, R² eine Alkylgruppe mit 6 bis 22 C-Atomen oder eine Alkoxymethyl- oder 2-Hydroxyalkoxymethylgruppe mit jeweils 6 bis 22 C-Atomen in der Alkoxygruppe ist, und wenn x = 1 ist, die Gruppe R² Wasserstoff ist und R³ eine der vorgenannten Bedeutungen von R² hat, wobei die Komponenten im Gewichtsverhältnis 9:1 bis 1:9 vorliegen. Die offenbarten antimikrobiell wirksamen Gemische werden als geeignet zur Herstellung antiseptisch wirksamer Hautreinigungsmittel und zur Konservierung wässriger Zubereitungen mikrobiell abbaubarer oder verderblicher Stoffe bezeichnet. Ein Hinweis auf eine Wirkung von 1,2-Decandiol gegenüber Keimen, die für die Bildung von Körpergeruch verantwortlich sind, ist in der EP 0 524 548 A1 nicht enthalten.

Eine Reihe weiterer Dokumente befasst sich entweder mit 1,2-Decandiol, ohne antimikrobielle Wirkungen dieser Substanz zu offenbaren, oder befasst sich mit der Wirkung anderer 1,2-Alkandiole ohne dabei 1,2-Decandiol zu betreffen. Hingewiesen sei insoweit auf die folgenden Dokumente: FR 2,755,371; JP 11 310506; WO 99/11237; WO 99/56715; WO 99/56716; JP 20 0044419; JP 11 335258; JP 10 053510; J. Food Sc. 42(3), 699-701; EP 1 000 542; DE 199 24 496 und JP 20 0148720.

Die vorliegende Erfindung betrifft primär ein Verfahren zur Hemmung des Wachstums von Körpergeruch verursachenden Keimen auf einem menschlichen oder tierischen Körper, wobei das Verfahren die topische Applikation von 1,2-Decandiol in einer Menge umfasst, die ausreicht, das Wachstum von Körpergeruch bildenden Keimen am Applikationsort zu hemmen. Die Körpergeruch verursachenden Keime sind hierbei ausgewählt aus Corynebacterium xerosis, Staphylococcus epidermidis, Brevibacterium epidermidis und Mischungen aus mindestens zwei der genannten Keime.

Üblicherweise wird 1,2-Decandiol in Form einer Formulierung appliziert werden, welche neben dem 1,2-Decandiol noch übliche Trägersubstanzen sowie beispielsweise einen Duftstoff oder dergleichen umfassen wird. In einem derartigen Verfahren wird eine 1,2-Decandiol umfassende Formulierung topisch so appliziert, dass die mit der Formulierung applizierte Menge an 1,2-Decandiol ausreicht, das Wachstum der Körpergeruch bildenden Keime am Applikationsort zu hemmen.

Im vorliegenden Zusammenhang besonders wichtig ist die Erkenntnis, dass eine antimikrobiell wirksame Formulierung, welche 1,2-Decandiol umfasst, kein Gel und insbesondere kein Glyceryl-Polymethacrylat-Gel umfassen muss, wie es in der US 6,123,953 offenbart ist.

Genauso wenig ist es notwendig, in einer solchen Formulierung einen Alkohol der Formel 1 vorzusehen, 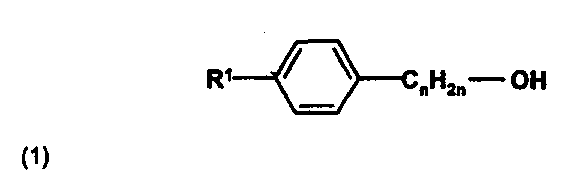 in der R¹ Wasserstoff oder eine Alkylgruppe mit 1 bis 4 C-Atomen und n eine ganze Zahl von 1 bis 6 ist, wie es in der EP 0 524 548 A1 offenbart ist.

Auch in Abwesenheit dieser synergistisch wirkenden Substanzen genügen äußerst geringe Mengen an 1,2-Decandiol, um das Wachstum der genannten Keime insoweit zu hemmen, dass es nicht mehr zu einer störenden Geruchsentwicklung kommt. Die applizierte Menge an 1,2-Decandiol wird dabei vorzugsweise so niedrig sein, dass das 1,2-Decandiol keine Hautirritationen hervorruft. Kosmetische Formulierungen, welche Hautirritationen hervorrufen können, sind von einer Verwendung im Markt nahezu ausgeschlossen, und deshalb wurde beispielsweise in der US 6,123,953 ein besonderer Wert darauf gelegt, dass in dem dort beschriebenen Gel-Diol-Substanzgemisch die Konzentration an Diol so gering sein kann, dass ein Hautirritationsrisiko nicht besteht (vgl. US 6,123,953, Spalte 2, Zeilen 9 bis 14).

Wenngleich 1,2-Decandiol gegenüber den genannten Keimen bereits für sich allein eine hervorragende Wirkung besitzt, ist es natürlich doch nicht ausgeschlossen. zur Bekämpfung dieser oder anderer Keime einen weiteren antimikrobiellen Wirkstoff zuzusetzen. Liegt neben 1,2-Decandiol in einer Formulierung noch ein weiterer antimikrobieller Wirkstoff vor, so ist die Formulierung vorzugsweise so zusammengesetzt, dass sie im Vergleich mit einer Vergleichsformulierung, die bei ansonsten gleicher Zusammensetzung kein 1,2-Decandiol umfasst, eine zumindest um den Faktor 4 erhöhte antimikrobielle Wirkung gegen *Corynebacterium xerosis, Staphylococcus epidermidis* und/oder *Brevibacterium epidermidis* besitzt: Der zweite antimikrobielle Wirkstoff wird also vorzugsweise nur wenig wirksam gegen die genannten Keime sein, aber eine hohe Wirksamkeit gegenüber anderen Keimen besitzen.

Es sei darauf hingewiesen, dass der Begriff 1,2-Decandiol im Rahmen des vorliegenden Textes sowohl (a) das 2S-konfigurierte Enantiomer als auch (b) das 2R-konfigurierte Enantiomer sowie (c) beliebige Mischungen aus 2S- und 2R-konfigurierten Decandiolen umfasst. Aus kommerziellen Gründen ist es zwar besonders vorteilhaft, die Razemate der jeweiligen Diole zur Bekämpfung der Körpergeruch bildenden Mikroorganismen einzusetzen, da diese synthetisch besonders leicht zugänglich sind, die reinen Enantiomere oder nicht-razemische Mischungen dieser Enantiomere sind aber ebenfalls für die erfindungsgemäßen Zwecke geeignet.

Mit Blick auf erfindungsgemäße Formulierungen, die 1,2-Decandiol umfassen, ist es bevorzugt, die Konzentration des Diols in diesen Formulierungen auf einen Wert zwischen 0,0002 und 20 Gew.-% einzustellen. Bevorzugt sind dabei Konzentrationen im Bereich zwischen 0,02 und 5 Gew.-%.

1,2-Decandiol besitzt nur einen sehr schwachen Geruch und kann deshalb auch in hoher Konzentration in Parfüm-Öl-Kompositionen eingesetzt werden. Derartige Parfüm-Öl-Kompositionen besitzen dann eine bakteriostatische Wirkung gegenüber Körpergeruch bildenden Keimen, so dass der Eigengeruch einer derartigen Parfüm-Öl-Komposition bei topischer Applikation länger erhalten bleibt. Da der Anteil eines Parfüms in einem kosmetischen Fertigprodukt meist nur etwa bei 1 Gew.-% liegt, wird ein Parfüm, welches 1,2-Decandiol als antimikrobiellen Wirkstoff enthält, vorzugsweise zu etwa 5 bis 50 Gew.-% aus 1,2-Decandiol bestehen. Ein Einsatz auch in derartig hohen relativen Konzentrationen ist unproblematisch möglich.

Die Erfindung wird nachfolgend anhand von Beispielen näher erläutert.

### Untersuchungen zur antimikrobiellen Wirkung von 1,2-Decandiolen

### 1. Allgemeine Testbedingungen (MHK-Wert-Messungen)

Der Nachweis der antimikrobiellen Wirkung von 1,2-Decandiol erfolgte mit Hilfe des Agardilutionsverfahren in Anlehnung an DIN 58 940/ICS und DIN 58 944/ICS. Es wurden Petrischalen von 5,5 cm Durchmesser mit 8,7 ml frisch hergestelltem und bei 50 °C flüssig gehaltenem Mueller-Hinton-Agar (Merck, Art. 1.05437 bzw. Wilkins-Chalgren-Agar-Boillon, Oxoid, Art. CM 643, supplementiert mit 10 g Agar-Agar/Liter) beschickt, denen die verschiedenen Konzentrationen der verdünnten Probe in 3,3 Vol.-% = 0,3 ml zugesetzt wurden.

2,6 ml der 3,3%-igen Proben wurden mit Ethanol (96%; Merck, Art. 1.00971) verdünnt. Durch fortlaufende 2:1-Verdünnung mit Ethanol (96%-ig) wurden die weiteren Testkonzentrationen der jeweiligen Verdünnungsreihen, die in Form geometrischer Reihen angelegt wurden, hergestellt.

Durch eine weitere Verdünnung mit dem Testagar (0,3 ml Probe bzw. entsprechender Verdünnungen + 8,7 ml Agar) wurden jeweils 30-fach niedrigere Endkonzentrationen erreicht (entspricht einer Anfangskonzentration von jeweils 1100 ppm). Die im folgenden angegebenen Konzentrationen beziehen sich auf die Reinsubstanz und sind in ppm umgerechnet. Pro Testkonzentration und Nährmedium wurden 2 Agarplatten gegossen.

Es wurden folgende Kontrollen mit jeweils 2 Agarplatten durchgeführt:

**Tabelle 1:**

| Kontrollen | |
|---|---|
| K1: 9,0 ml Mueller-Hinton-Agar | (unbeimpft) |
| K2: 8,7 ml Mueller-Hinton-Agar + 0,3 ml Ethanol (96%) | (unbeimpft) |
| K3: 8,7 ml Mueller-Hinton-Agar + 0,3 ml Ethanol (96%) | (beimpft) |
| K4: 9,0 ml Mueller-Hinton-Agar | (beimpft) |

Nach Verfestigung und Trocknung (ca. 1 h bei 37 °C) wurden die Testplatten punktförmig mit jeweils 1 µl der nachfolgend aufgeführten Testkeimsuspensionen beimpft. Zur Überprüfung von Reinheit und Identität wurden die aerob wachsenden Bakterien *Brevibacterium epidermidis, Corynebacterium xerosis; Staphylococcus epidermidis* auf Columbia Blut-Agar (BioMérieux, Art. 43049) kultiviert. Weitere Angaben zu den Testkeimen sind Tabelle 2 zu entnehmen.

**Tabelle 2:**

| Testkeime (Stammbezeichnungen) | |
|---|---|
| Testkeim | Stammbez. |
| | |
| *Brevibacterium epidermidis* | ATCC 35514 |
| *Corynebacterium xerosis* | ATCC 7711 |
| *Staphylococcus epidermidis* | ATCC12228 |

Die Herstellung der Testkeimsuspensionen der Testkeime für *Brevibacterium epidrmidis, Corynebacterium xerosis*, und *Staphylococcus epidermidis* erfolgte durch Bebrütung von Mueller-Hinton-Bouillon (Merck, Art. 1.10293) bei 36 °C, die mit wenigen Einzelkolonien der jeweiligen Testkeime beimpft worden war. Nach dem Erreichen einer deutlichen Trübung wurde den Suspensionen so viel sterile Nährbouillon zugegeben, daß deren Trübung dem McFarland Standard 0,5 entsprach (ca. 10⁸ KBE/ml).

Die inokulierten Platten wurden unter den in Tabelle 3 angegebenen Bedingungen bebrütet und anschließend ausgewertet. Als MHK (Minimale Hemmkonzentration) wurde die niedrigste Wirkstoffkonzentration angesehen, bei der makroskopisch kein Wachstum vorhanden ist. Minimales, kaum sichtbares Wachstum oder wenige kleine Einzelkolonien wurden als Hemmung bewertet.

**Tabelle 3:**

| Inokulation und Bebrütung | | | | |
|---|---|---|---|---|
| Testkeim | Stamm-Bez. | Wachstums- | Nährmedium | Bebrütung |
| | | bedingungen | | |
| | | | | |
| ***Brevibacterium epidermidis*** | **ATCC 35514** | **Aerob** | **Mueller-Hinton-Agar** | **18h bei 36 °C** |
| ***Corynebacterium xerosis*** | **ATCC 7711** | **aerob** | **Mueller-Hinton-Agar** | **18h bei 36 °C** |
| ***Staphylococcus epidermidis*** | **ATCC 12228** | **aerob** | **Mueller-Hinton-Agar** | **18h bei 36 °C** |

### 2. MHK-Werte für 1,2-Decandiol im Vergleich mit 1,2-Octandiol

Gemäß den unter 1. beschriebenen allgemeinen Testbedingungen wurden Suspensionen der oben beschriebenen Mikroorganismen bebrütet und die MHK-Werte von 1,2-Decandiol bestimmt (vgl. Tabelle 4). Zu Referenzzwecken wurden ebenfalls die MHK-Werte für 1,2-Octandiol ermittelt.

**Tabelle 4:**

| MHK-Werte [ppm] für 1,2-Octandiol und 1,2-Decandiol | | | |
|---|---|---|---|
| Testkeim | Stamm-Bez. | 1,2-Octandiol | 1,2-Decandiol |
| | | | |
| *Staphylococcus epidermidis* | ATCC 12228 | 1800 | 225 |
| *Corynebacterium xerosis* | ATCC 7711 | 3600 | 225 |
| *Brevibacterium epidermidis* | ATCC 35514 | 3600 | 450 |

Der Vergleich der 1,2-Diole zeigt, dass die MHK-Werte für 1,2-Octandiol zwischen 3600 und 1800 ppm liegen und somit relativ hoch sind. Im Gegensatz hierzu liegen die MHK-Werte für 1,2-Decandiol deutlich niedriger.

### 3. Wachstumskurve für Corynebacterium xerosis - Allgemeine Testbedingungen

Eingesetzte Lösungen:
AC-Medium: 37g Brain Heart Infusion, 5g Glucose, 1ml Tween 80 ad 1l H2O

Testsubstanzen:
1,2-Octandiol (0,3%)
1,2-Decandiol (0,3%)
Famesol (0,3%)

Kontrolle:
AC-Medium ohne Wirkstoff

Testlösungen:
Jeweilige Testsubstanz in AC-Medium

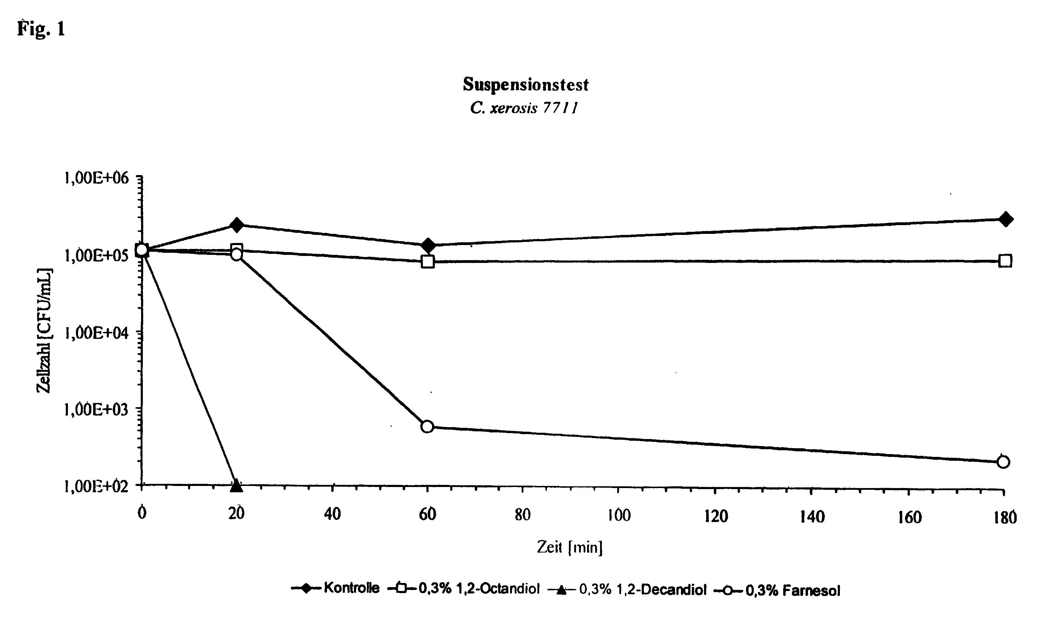

### Suspensionstest (vgl. Fig. 1)

60 ml AC-Medium wurden mit 0,4 ml einer frischen Übemachtkultur des Testorganismus *Corynebacterium xerosis* (*C*. *xerosis* 7711) beimpft. Das Kulturgefäß wurde bei 30°C mit 250 Upm geschüttelt, bis eine Zelldichte von 10⁵ bis 10⁶ Zellen/ml erreicht war. Die Testsubstanzen wurden eingewogen und jeweils in 2,5 ml AC-Medium unter kurzem Erwärmen auf 60°C gelöst. Anschließend wurden je 2,5ml Bakteriensuspension mit den so gewonnenen Testlösungen versetzt und unter Beibehaltung der Wachstumsbedingungen 20, 60 und 180 min inkubiert. Sodann wurden 100µl der inkubierten Testlösungen entnommen und der Bakterientiter duch Ausplattieren auf Nährmedium bestimmt.

In Figur 1 sind die Wachstumskurven für die vorstehend beschriebenen Versuchsansätze aufgezeigt.

### 4. Ergebnisse (Wachstumskurven Corynebacterium xerosis)

Die Wachstumskurve für *Corynebacterium xerosis* (Fig. 1) zeigt, dass bei einer Einsatzkonzentration von 0,3% 1,2-Decandiol innerhalb der getesteten Substanzen die bei weitem stärkste Wirksamkeit besitzt. Die Wirksamkeit des 1,2-Decandiols ist sogar größer als die des Famesols, eines bekannten Deowirkstoffs (siehe oben), der als Positivkontrolle verwendet wurde.

## Patentansprüche

1. Verfahren zur Hemmung des Wachstums von Körpergeruch verursachenden Keimen auf einem menschlichen oder tierischen Körper, mit folgendem Schritt:
- topische Applikation von 1,2-Decandiol,
wobei die applizierte Menge an 1,2-Decandiol ausreicht, das Wachstum von Körpergeruch bildenden Keimen am Applikationsort zu hemmen,
wobei die Körpergeruch verursachenden Keime ausgewählt sind aus der Gruppe, die aus
- *Corynebacterium xerosis*,
- *Staphylococcus epidermidis*,
- *Brevibacterium epidermidis* und
- Mischungen aus zwei oder mehr der vorgenannten Keime
besteht.

2. Verfahren zur Hemmung des Wachstums von Körpergeruch verursachenden Keimen auf einem menschlichen oder tierischen Körper, mit folgendem Schritt:
- topische Applikation einer 1,2-Decandiol umfassenden Formulierung,
wobei die mit der Formulierung applizierte Menge an 1,2-Decandiol ausreicht, das Wachstum von Körpergeruch bildenden Keimen am Applikationsort zu hemmen,
wobei die Körpergeruch verursachenden Keime ausgewählt sind aus der Gruppe, die aus
- *Corynebacterium xerosis*,
- *Staphylococcus epidermidis*,
- *Brevibacterium epidermidis* und
- Mischungen aus zwei oder mehr der vorgenannten Keime
besteht.

3. Verfahren nach Anspruch 2, **dadurch gekennzeichnet, dass** die Formulierung kein Glyceryl-Polymethacrylat-Gel und vorzugsweise überhaupt kein Gel umfasst.

4. Verfahren nach einem der Ansprüche 2 oder 3, **dadurch gekennzeichnet, dass** die Formulierung keinen Alkohol der Formel 1 umfasst, 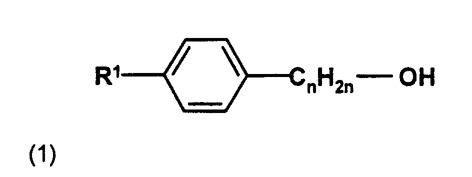 in der R¹ Wasserstoff oder eine Alkylgruppe mit 1 bis 4 C-Atomen und n eine ganze Zahl von 1 bis 6 ist

5. Verfahren nach einem der vorangehenden Ansprüche, wobei die applizierte Menge an 1,2-Decandiol so niedrig ist, dass das 1,2-Decandiol keine Hautirritation hervorruft.

6. Verwendung von 1,2-Decandiol als antimikrobieller Wirkstoff gegen *Corynebacterium xerosis, Staphylococcus epidermidis* und *Brevibacterium epidermidis.*

## Claims

1. Method for inhibiting the growth of organisms causing body odour on a human or animal body, having the following step:
- topical application of 1,2-decanediol,
wherein the amount of 1,2-decanediol applied is sufficient to inhibit the growth of organisms forming body odour at the site of application,
wherein the organisms causing body odour have been selected from the group that consists of:
- *Corynebacterium xerosis*,
- *Staphylococcus epidermidis*,
- *Brevibacterium epidermidis* and
- mixtures of two or more of the abovementioned organisms.

2. Method for inhibiting the growth of organisms causing body odour on a human or animal body, having the following step:
- topical application of a formulation containing 1,2-decanediol, wherein the amount of 1,2-decanediol applied by means of the formulation is sufficient to inhibit the growth of organisms forming body odour at the site of application,
wherein the organisms causing body odour have been selected from the group that consists of:
- *Corynebacterium xerosis,*
- *Staphylococcus epidermidis,*
- *Brevibacterium epidermidis* and
- mixtures of two or more of the abovementioned organisms.

3. Method according to Claim 2, **characterised in that** the formulation does not contain glyceryl polymethacrylate gel and preferably does not contain any gel at all.

4. Method according to one of Claims 2 or 3, **characterised in that** the formulation does not contain an alcohol of the formula 1. 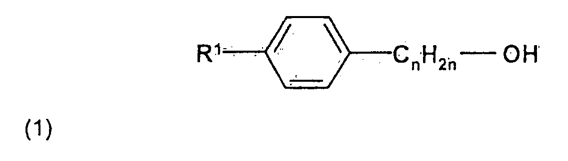 in which R¹ is hydrogen or an alkyl, group having 1 to 4 C atoms and n is an integer from 1 to 6.

5. Method according to one of the preceding claims, wherein the amount of 1,2-decanediol applied is so small that the 1,2-decanediol does not give rise to any skin irritation.

6. Use of 1,2-decanediol as an antimicrobial active compound against *Corynebacterium xerosis, Staphylococcus epidermidis* and *Brevibacterium epidermidis*.

## Revendications

1. Procédé pour l'inhibition de la croissance de germes responsables d'odeurs corporelles sur un corps humain ou animal, avec l'étape suivante :
- application topique de décanediol-1,2,
moyennant quoi la quantité appliquée en décanediol-1,2 suffit à inhiber la croissance des germes responsables d'odeurs corporelles sur le lieu d'application,
moyennant quoi les germes responsables d'odeurs corporelles sont choisis dans le groupe comprenant :
- *corynebacterium xerosis*,
- *staphylococcus epidermidis*,
- *brevibacterium epidermidis* et
- des mélanges de deux ou plus des germes cités précédemment.

2. Procédé pour l'inhibition de la croissance de germes responsables d'odeurs corporelles sur un corps humain ou animal, avec l'étape suivante :
- application topique d'une formulation comprenant du décanediol-1,2, moyennant quoi la quantité appliquée en décanediol-1,2 avec la formulation suffit à inhiber la croissance des germes responsables d'odeurs corporelles sur le lieu d'application,
moyennant quoi les germes responsables d'odeurs corporelles sont choisis dans le groupe comprenant :
- *corynebacterium xerosis*,
- *staphylococcus epidermidis*,
- *brevibacterium epidermidis* et
- des mélanges de deux ou plus des germes cités précédemment.

3. Procédé selon la revendication 2, **caractérisé en ce que** la formulation ne comprend pas de gel polyméthacrylate de glycéryle, et de préférence absolument aucun gel.

4. Procédé selon l'une quelconque des revendications 2 ou 3, **caractérisé en ce que** la formulation ne contient aucun alcool de formule 1, 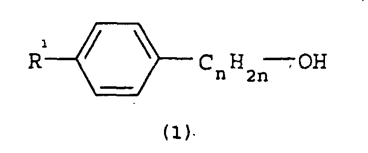 dans lequel R¹ représente un hydrogène ou un groupe alkyle avec 1 à 4 atomes C et n un nombre entier de 1 à 6.

5. Procédé selon l'une quelconque des revendications précédentes, moyennant quoi la quantité appliquée en décanediol-1,2 est si faible que le décanediol-1,2 n'entraîne aucune irritation de la peau.

6. Utilisation du décanediol-1,2 comme agent antimicrobien contre *corynehacterium xerosis, staphylococcus epidermidis et brevibacterium epidermidis*.
